# EUROPEAN PATENT APPLICATION

(11) **EP 4 059 526 A1**
(43) Date of publication of application: **21.09.2022**
(21) Application number: 22160839.1
(22) Date of filing: 08.03.2022
(51) Int. Cl.: A61L 9/20, A41D 13/11

(54) **A DEVICE FOR PROTECTION OF UPPER RESPIRATORY TRACT**

(30) Priority: 17.03.2021 CZ 202138647 U
(71) Applicant: EMP - Centauri, Spolecnost S Rucením Omezenym, 339 01 Klatovy (CZ)
(72) Inventor: Vána, Marian, 34021 Strázov (CZ); Vána, Richard, 34021 Strázov (CZ)
(74) Representative: Sedlák, Jirí

(57) **Abstract**

The device (14) for protection of upper respiratory tract comprises at least one base body (15) comprising at least one main air chamber (5), and at least one ambient air inlet (6), and further comprising at least one fastening means (4). The base body (15) further comprises at least one inlet disinfection chamber (2). This disinfection chamber (2) is provided with at least one ambient air inlet (6), at least one main source (10) of UV radiation and at least one ambient air outlet (16). The ambient air outlet (16) of the disinfection chamber (2) is connected to the main air chamber (5) via a main fan (3). The air chamber (5) is provided with at least one main exhalation opening (19) for exhausting air from the device (14). This arrangement ensures that the protected person can breathe easily while maintaining a clean and disinfected internal environment of the device (14). Furthermore, the device (14) provides perfect isolation from the surrounding atmosphere with the possible presence of harmful bacteria and viruses.

## Description

### Field of the Invention

The invention relates to the field of medical devices, in particular to devices for protection of the upper respiratory tract.

### Background of the Invention

At present, a variety of equipment and protective devices are used to protect the upper respiratory tract. These include various types of respirators, surgical masks, face and breathing masks, respirators with active filters, etc. Surgical masks or drapes are designed to be worn over the mouth and nose to trap bacteria in the droplets and aerosols coming out of the wearer's mouth and nose. They are not designed to protect the wearer from inhaling airborne bacteria or viral particles and are less effective than respirators providing better protection due to their material, shape and tightness. Nevertheless, surgical drapes are better than homemade improvised cloth drapes, which can also serve for basic protection of upper respiratory tract. The correct use of each device for protection of the upper respiratory tract also matters. For example, failure to fit the drape to the face due to an unshaven face reduces its effectiveness.

Usually surgical masks are three-layered, this three-layer material is made of nonwoven fabric. Most surgical masks are crinkled or have folds. Three folds are used to allow the wearer to extend the mask to cover the area from the nose to the chin. Typical three-layered surgical masks have a top edge with double stitching that is designed to cover the nose, and a metal wire is integrated inside so that the mask can be securely shaped around the nose.

Another well-known device for protection of the upper respiratory tract is the respirator, which provides the wearer with better protection than surgical masks. A respirator or half mask strongly protects against the penetration of viruses and bacteria from the external environment of its wearer. The half mask covers the nose, mouth and chin, and may have an opening in the front, i.e. an inhalation and/or exhalation valve. The half mask with an exhalation valve protects the wearer from the penetration of viruses and bacteria from the external environment, but does not protect the environment from the wearer because it does not filter the exhaled air. Thus, what the wearer of this ventilator exhales is not filtered in any way.

Another well-known device for protection of upper respiratory tract are protective face shields against droplets and liquid splashes, which are made of plastic material and can therefore be disinfected and used repeatedly. In combination with a drape or respirator, they provide a perfect protection. Face shields should therefore be used to supplement other device for protection of the upper respiratory tract, not as a stand-alone protective device.

These devices for protection of the upper respiratory tract, i.e. aids and protective equipment, are usually attached to the face by means of straps, rubber loops, textile cords. In any case, these protective devices and equipment are always designed to be firmly attached to the face and to minimise or eliminate air gaps between the face and the protective device or equipment. However, this makes breathing difficult for the protected person, causing the interior of the protective device or equipment to become stuffy and clogged with dirt, live bacteria and viruses or saliva. After prolonged use, such protective equipment and devices become very uncomfortable and unhygienic. A prerequisite for the correct functioning of these types of protective equipment is that they fit perfectly to the face of the protected person, but this is difficult in practice. This fact often makes them unreliable or reduces their effectiveness.

It is the object of the invention to create a device for protection of the upper respiratory tract which would eliminate the shortcomings of known solutions, which would ensure easy breathing of the protected person, maintenance of a clean and disinfected internal environment of the device, perfect isolation from the surrounding atmosphere with the possible occurrence of harmful bacteria and viruses, and which could be used comfortably and for a long time with guaranteed and stable effectiveness.

### The summary of the invention

This object is resolved by the device for protection of the upper respiratory tract according to the present invention. The device comprises at least one base body comprising at least one main air chamber formed between the device and the user's face with at least one ambient air inlet and at least one fastening means for fastening the device to the user's face.

The subject-matter of the invention consists in the fact that the device uses disinfection of the inhaled air by means of UV radiation and at the same time uses its forced suction from the surrounding atmosphere into the upper respiratory tract by means of a fan blowing air into the upper respiratory tract. The base body is formed from a plastic suitable for 3D printing, such as polylactide filaments or PLA, acrylonitrile butadiene styrene or ABS, nylon, etc., and further includes at least one inlet disinfection chamber provided with at least one ambient air inlet, at least one main source of UV radiation, and at least one ambient air outlet. The ambient air outlet of the disinfection chamber is connected via a main fan to the main air chamber having at least one main air outlet for exhausting air from the device. Thus, the device makes use of the atmospheric overpressure generated by the fan in the area in front of the face before entering the upper respiratory tract. When the ventilator is in operation, atmospheric overpressure is created in the main air chamber to prevent penetration of dirt and microorganisms from the surrounding atmosphere without passing first through the ventilator and being disinfected in the disinfection chamber by means of an LED-type emitting diode.

The above-described device protects a in particular the person wearing the device for protection of the upper respiratory tract according to the present invention, but it does not provide any practical protection to persons in the nearby the person wearing this device, since the exhaled air mixes with the aspirated air and diffuses through the exhalation opening to exhaust the air from the device into the surrounding atmosphere. The device, which also addresses the protection of these persons, has, in addition, in a preferred embodiment, a main air chamber provided with an internal exhalation chamber with an outlet disinfection chamber. This outlet disinfection chamber is provided with at least one auxiliary source of UV radiation and an auxiliary exhalation opening for exhausting air from the device, ensuring free passage of disinfected exhaled air into the ambient atmosphere. To facilitate removal of exhaled air from the device, an auxiliary fan may preferably be placed on the auxiliary exhalation opening and provided with at least one secondary exhalation opening for exhausting disinfected exhaled air to the ambient atmosphere. The power of the auxiliary fan is lesser than the power of the main fan, so that the volume of air forced through the auxiliary fan is less per unit time compared to the main fan.

In an advantageous embodiment, the main fan, the auxiliary fan, the main source of UV radiation and the auxiliary source of UV radiation are connected to a box with a printed circuit board and control electronics having at least one voltage source, which is ideally a low or low voltage source that supplies them. The voltage source is a battery or a battery pack.

The apparatus preferably further comprises a front face plate in which at least one ambient air inlet is arranged.

The main UV radiation source and the auxiliary UV radiation source consist of at least one LED type diode with an emission characteristic in the ultraviolet region of the spectrum of 100 to 380 nm, thus ensuring disinfection of the inhaled and exhaled air.

In a preferred arrangement, the fastening means is formed as a pair of shaped wires or shaped tubes or rubber bands or textile straps. To fix the device to the head of the protected person, the ends are suitably shaped or the device is additionally provided with a support firmly connected thereto and designed to rest against a part of the head or face, for example the chin. There is thus a free area between the protected face and the device which is directly connected to the surrounding atmosphere and through which air can flow forcibly from the main ventilator to the upper respiratory tract area and then along the face to the surrounding atmosphere, but also into the respiratory tract. A further advantageous arrangement of the fastening means is the use of a fastening band around the head to which the base body is fixed.

The device basically does not touch the face at all, because it does not have to touch the face at all and it is more comfortable for the user, the device only touches the head at the point of contact of the fastening means with the head or possibly only with a small part of the face at the bottom of the face. The base body itself, specifically the end of the main air chamber, which in fact is the main exhalation opening, is typically fixed by the fastening means a few millimetres to about a centimetre in front of the face. The main exhalation opening extends in front of the face in the area of the nose and mouth and forms the end of the main air chamber.

The advantages of the device for protection of the upper respiratory tract according to the present invention consist in particular in that it ensures easy breathing of the protected person, maintains a clean and disinfected internal environment of the device, in perfect isolation from the ambient atmosphere where harmful bacteria and viruses can occur, in that it can be used comfortably and for a long time with a guaranteed and stable effectiveness, and, in addition, it eliminates the need for any seals as well as air filters and their replacement.

### Brief description of the drawings

The present invention will be explained in more detail in the following illustrations, where:
- Figure 1: shows a front view of the device for protection of upper respiratory tract,
- Figure 2: shows a cross-section of the device for protection of the upper respiratory tract,
- Figure 3: shows a cross-section of the device for protection of the upper respiratory tract with an internal exhalation chamber,
- Figure 4: shows a cross-section of the device for protection of the upper respiratory tract with an internal exhalation chamber and with an auxiliary fan,

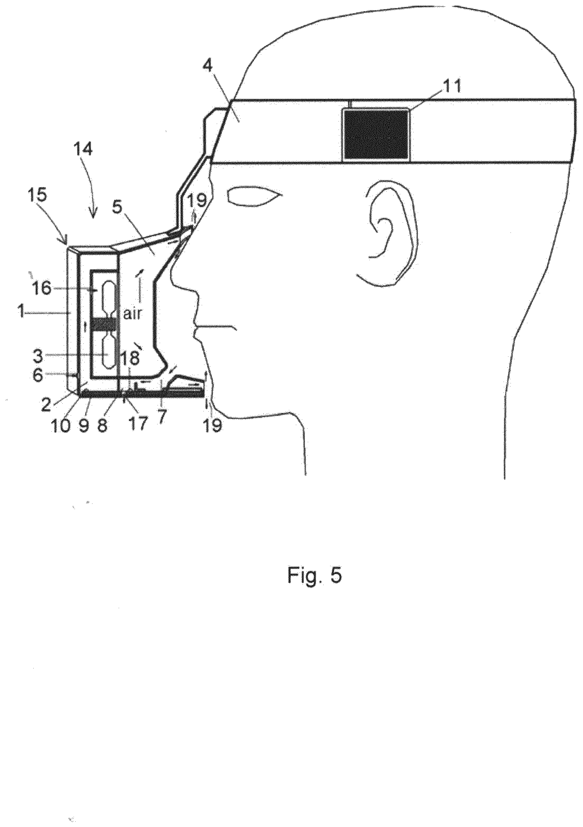

- Figure 5: shows a cross-section of the device for protection of the upper respiratory tract with a belt as a fastening means.

### Preferred embodiments of the invention

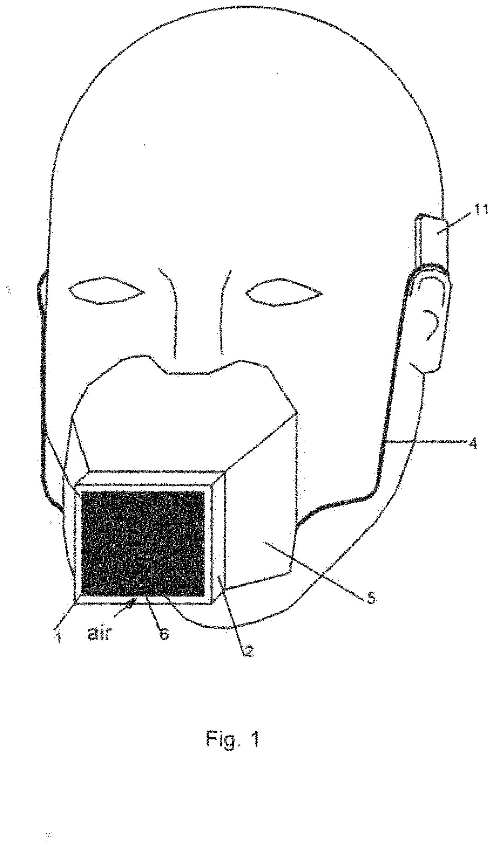

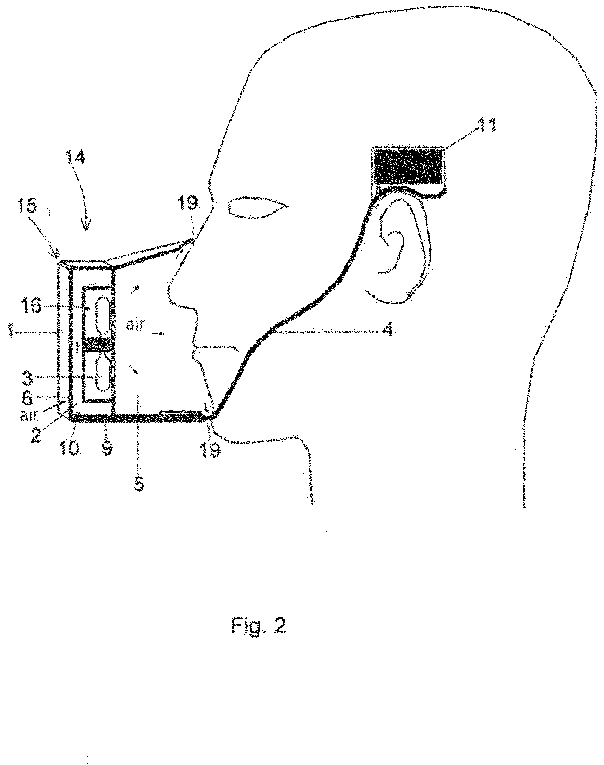

The device 14 for protection of the upper respiratory tract according to the present invention, shown in front view in Figure 1, comprises a base body 15 of plastic, made of polylactide fibers, which comprises a front face plate 1 with an opening forming an ambient air inlet 6 leading to an inlet disinfection chamber 2, wherein the inlet disinfection chamber 2 is rigidly attached to the front face plate 1. In another example a preferred embodiment, the base body 15 is formed from another plastic material suitable for 3D printing, namely acrylonitrile butadiene styrene or ABS or nylon. The front face plate 1 is made of an aluminium sheet. As shown in the cross-section in Figure 2, the inlet disinfection chamber 2 has a main fan 3 fixedly connected to the ambient air outlet 16, powered by a voltage source 11, being the source of extra low or low voltage which in this case is a battery. In another example of a preferred embodiment, a battery pack may be used as the voltage source 11.

When the main fan 3 is in operation, the device 14 draws air from the ambient atmosphere through an opening of the ambient air inlet 6 in the front face plate 1 into the inlet disinfection chamber 2 and from its ambient air outlet 16 the air passes through the main fan 3 itself. The inlet disinfection chamber 2 houses the main source 10 of UV radiation, namely a LED type diode with an emission characteristic in the ultraviolet region of the spectrum of 100 to 380 nm, and this diode is designed to irradiate all the forced air drawn into the inlet disinfection chamber 2. The drawn air from the ambient atmosphere brings bacteria and viruses into the inlet disinfection chamber 2, which are destroyed with the help of the UV radiation, and the drawn air is thus disinfected in the inlet disinfection chamber 2. The main fan 3 blows the disinfected air into the main air chamber 5, whereby the disinfected air flows into the upper respiratory tract area, the main air chamber 5 having dimensions corresponding to the coverage of the mouth and nose area of the human face. These dimensions are 7,5 x 7,5 cm.

The main air chamber 5, when placed in front of the user's face in the nose and chin area, is provided a main exhalation opening 19 covering the entire nose, mouth and chin area. The voltage source 11, the electronic circuitry fed from this voltage source 11 to control the speed of the main fan 3 and to control the emission of the main source 10 of UV radiation are connected to a box 9 with the printed circuit board and the control electronics, and together form a solid and functional unit.

There are two end pieces attached to the device 14 forming fastening means 4 designed to fit behind the ears of the protected person and to rest against part of the face or head. The ends of the fastening means 4 are formed from suitably shaped wires or profiles, and these are shaped so that the orifice of the main air chamber 5 ends in front of the face without the need to touch it. In another example of a preferred embodiment, the fastening means 4 may be formed to be secured behind the ears of the user by other means, such as securing the entire device 14 behind the ears or to the head by means of a fastening strap, rubber bands, straps, textile cord, etc.

The surface of the inner walls of the inlet disinfection chamber 2 can be constructed of a material with good reflectivity in the UVC region, which in this case is an aluminium foil. In another example of a preferred embodiment, the material with good UVC reflectivity is an expanded polytetrafluoroethylene or ePTFE film.

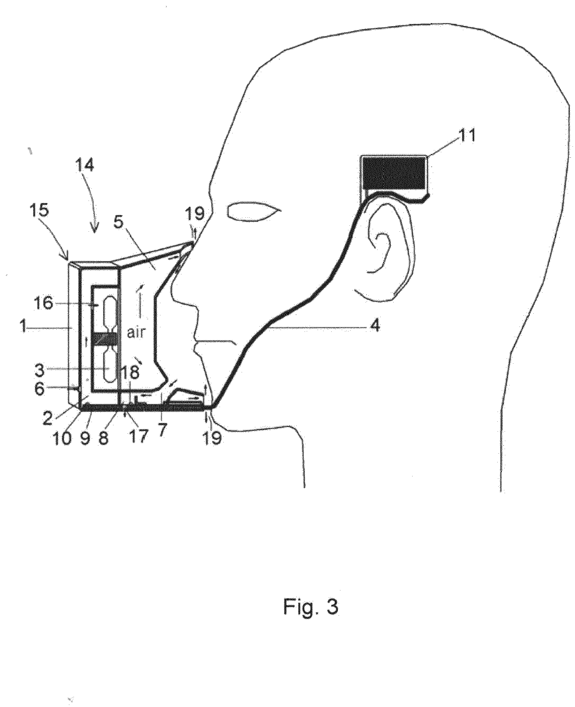

Figure 3 shows a section of the device 14, which is further provided with an internal exhalation chamber 7 located in the main air chamber 5. The internal exhalation chamber 7 opens into an outlet disinfection chamber 8 provided with an auxiliary exhalation opening 17 for the free passage of the exhaled air into the surrounding atmosphere. The outlet disinfection chamber 8 is equipped with an auxiliary source 18 of UV radiation, which is also formed by LEDs with an emission characteristic in the ultraviolet spectral region of 100 to 380 nm. This diode is designed to irradiate all exhaled air from the outlet disinfection chamber 8. The air entering the device 14 through the ambient air inlet 6 is, after disinfection in the inlet disinfection chamber 2, blown by the main fan 3 into the main air chamber 5 where it is drawn in through the upper respiratory track of the user wearing the device 14. The exhaled air then enters the internal exhalation chamber 7 which then passes into the outlet disinfection chamber 8 where the exhaled air is disinfected by UV radiation. The exhaled air leaves the device 14 through the auxiliary exhalation opening 17.

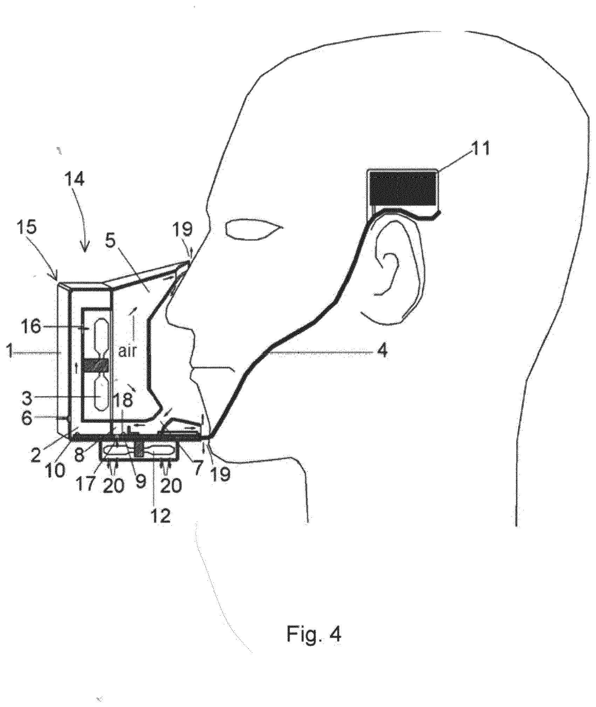

Figure 4 shows a section of the device 14 which is further equipped with an auxiliary fan 12 for easier exhaust air removal, in the area of the auxiliary exhalation opening 17, i.e. in the area of the outlet of the air from the outlet disinfection chamber 8. The auxiliary fan 12 is provided with four secondary exhalation openings 20 through which the exhaled disinfected air leaves the device 14. A power source 11 and an electronic circuit powered by this power source 11 are also provided for controlling the speed of the auxiliary fan 12 and for controlling the emission of the auxiliary source 18 of UV radiation and are connected to box 9 the printed circuit board and the control electronics and together form a solid and functional unit.

### Industrial applicability

The device for protection of the upper respiratory tract according to the present invention can be used as a protective medical device, protecting the user against viruses and bacteria in particular.

### List of relationship tags

- 1: front face plate
- 2: inlet disinfection chamber
- 3: main fan
- 4: fastening means
- 5: main air chamber
- 6: ambient air inlet
- 7: internal exhalation chamber
- 8: outlet disinfection chamber
- 9: box with printed circuit board and control electronics
- 10: the main source of UV radiation
- 11: voltage source
- 12: auxiliary fan
- 14: the device
- 15: base body
- 16: ambient air outlet
- 17: auxiliary exhalation opening
- 18: auxiliary source of UV radiation
- 19: main exhalation opening
- 20: secondary exhalation opening

## Claims

1. A device (14) for protection of the upper respiratory tract comprising at least one base body (15) comprising at least one main air chamber (5), at least one ambient air inlet (6) and at least one fastening means (4), **characterized in that** the base body (15) further comprises at least one inlet disinfection chamber (2) provided with at least one ambient air inlet (6), at least one main source (10) of UV radiation and at least one ambient air outlet (16), wherein the ambient air outlet (16) of the disinfection chamber (2) is connected via a main fan (3) to a main air chamber (5) which is provided with at least one main exhalation opening (19) for exhausting air from the device (14).

2. The device (14) according to claim 1, **characterized in that** the main air chamber (5) is provided with an internal exhalation chamber (7) having an outlet disinfection chamber (8), the outlet disinfection chamber (8) being provided with at least one auxiliary source (18) of UV radiation and an auxiliary exhalation opening (17) for exhausting air from the device (14).

3. The apparatus (14) according to claim 2, **characterized in that** an auxiliary fan (12) is connected to the auxiliary exhalation opening (17), which is provided with at least one secondary exhalation opening (20).

4. The device (14) according to claim 3, **characterized in that** the power of the auxiliary fan (12) is lesser than the power of the main fan (3).

5. The device (14) according to any one of claims 1 to 4, **characterized in that the** main fan (3), the auxiliary fan (12), the main source (10) of UV radiation and the auxiliary source (18) of UV radiation are connected to a box (9) with a printed circuit board and control electronics, which is provided with at least one voltage source (11).

6. The device (14) according to claim 5, **characterized in that** the voltage source (11) is a rechargeable battery or battery pack.

7. The device (14) according to any one of claims 1 to 6, **characterized in that** it further comprises a front face plate (1) in which at least one ambient air inlet (6) is arranged.

8. The device (14) according to any one of claims 1 to 7, **characterized in that** the main source (10) of UV radiation and the auxiliary source (18) of UV radiation is made of at least one LED-type diode having an emission characteristic in the ultraviolet region of the spectrum of 100 to 380 nm.

9. Device (14) according to any one of claims 1 to 8, **characterized in that** the fastening means (4) is formed as a pair of shaped wires or shaped tubes or rubber bands or textile straps or as a fastening strip.
